# EUROPÄISCHE PATENTSCHRIFT

(11) **EP 3 782 096 B1**
(45) Veröffentlichungstag und Bekanntmachung des Hinweises auf die Patenterteilung: **07.06.2023**
(21) Anmeldenummer: 19740500.4
(22) Anmeldetag: 04.07.2019
(51) Int. Cl.: G06Q 10/06, G06Q 50/26, G06Q 50/22, G16H 10/00, G16H 20/10, A61B 5/00, G16H 40/63

(54) **ORTSUNABHÄNGIGE EINNAHMEKONTROLLE**
LOCATION-INDEPENDENT INTAKE CONTROL
CONTRÔLE DE PRISE RÉALISÉ INDÉPENDAMMENT DU LIEU

(43) Veröffentlichungstag der Anmeldung: 24.02.2021
(73) Patentinhaber: Ruma GmbH, 50933 Köln (DE)
(72) Erfinder: WETZKE, Monika, 51375 Leverkusen (DE)
(74) Vertreter: Richardt Patentanwälte PartG mbB
(86) Internationale Anmeldenummer: PCT/EP2019/067956
(87) Internationale Veröffentlichungsnummer: WO 2021/001043

(56) Entgegenhaltungen:
- EP-A1- 1 410 014
- WO-A1-2018/037148
- DE-A1-102017 220 500
- US-A1- 2019 035 499
- US-A1- 2019 080 791
- US-A1- 2019 198 144
- THATCHER CAMDEN ET AL: "Pharmaceutical uses of Blockchain Technology", 2018 IEEE INTERNATIONAL CONFERENCE ON ADVANCED NETWORKS AND TELECOMMUNICATIONS SYSTEMS (ANTS), IEEE, 16. Dezember 2018 (2018-12-16), Seiten 1-6, XP033547215, DOI: 10.1109/ANTS.2018.8710154 [gefunden am 2019-05-08]

## Beschreibung

Die Erfindung bezieht sich auf das Feld des Gesundheitswesens.

Insbesondere bezieht sich die Erfindung auf die Kontrolle der Einnahme einer bestimmten pharmazeutischen Zusammensetzung.

CN108538355 A beschreibt eine Vorrichtung und ein Verfahren zur Sicherstellung, dass ein Patient das Medikament eingenommen hat. Die Vorrichtung zur Sicherstellung der Medikamenteneinnahme umfasst eine Videoaufnahmevorrichtung, eine Speicher- und eine Verarbeitungsvorrichtung. Die Videoaufnahmevorrichtung ist so konfiguriert, dass sie eine oder mehrere Videosequenzen speichert. Die Verarbeitungsvorrichtung kann die Videosequenzen analysieren, um verdächtige Aktivitäten des Benutzers zu bestimmen. Die Verarbeitungsvorrichtung ist so konfiguriert, dass sie passende Arzneimittelverpackungen erkennen kann.

DE 10 2014 007249 A1 beschreibt eine Vorrichtung, die zur Personenerkennung, zur Erinnerung und/oder zur Zeitfestlegung von Verabreichungsfristen dienen kann. Darüber hinaus ermöglicht die Vorrichtung die Kontrolle der Medikamenteneinnahme und die Überprüfung, ob der Benutzer das Arzneimittel durch Übergeben ausgeschieden hat.

Die vorliegende Erfindung schlägt ein Verfahren vor, das ermöglicht, den Weg einer pharmazeutischen Zusammensetzung von der Herstellung bis zur Patienteneinnahme nachzuvollziehen.

### DEFINITIONEN

### Pharmazeutische Zusammensetzung (pZ)

Eine pharmazeutische Zusammensetzung (pZ) im Sinne der Erfindung ist eine Zusammensetzung, die einem Patienten im Rahmen einer therapeutischen Behandlung, oder im Rahmen eines diagnostischen Verfahrens verabreicht wird. Eine pZ kann daher Wirkstoffe zur Behandlung von Krankheiten enthalten. Die pZ kann aber auch lediglich nicht metabolisierbare Substanzen zur Authentifizierung der biologischen Proben des Patienten und keine Wirkstoffe enthalten. Die pZ kann allerdings sowohl einen Wirkstoff, als auch nicht metabolisierbare Substanzen zur Authentifizierung der biologischen Proben des Patienten enthalten.

Die pZ kann für beliebige Applikationsformen, wie z.B. für die orale, parenterale, enterale oder perkutane Verwendung, geeignet sein. Die pZ kann in beliebiger Form, wie z.B. als Pulver, Lösung, Tablette, Kapsel oder Fertigspritze, vorliegen.

Mit der pZ ist eine generische pharmazeutische Zusammensetzung gemeint, die eine definierte Zusammensetzung aufweist. Eine einzelne pZ bezeichnet eine einzeln verpackte pZ.

### Behältnis

Ein Behältnis im Sinne der Erfindung ist ein Gegenstand, in dem sich die pZ befindet.

Das Behältnis kann jedwede Art der Umhüllung sein, die die pZ unmittelbar umschließt. Das Behältnis kann beispielsweise ein Fläschchen, eine Blisterpackung, eine Spritze, eine Tüte oder Ähnliches sein.

### Äußere Verpackung

Das erfindungsgemäße Behältnis befindet sich in einer äußeren Verpackung.

Die äußere Verpackung beinhaltet das erfindungsgemäße Behältnis und ggf. auch weitere Informationen, wie z. B. Gebrauchsanweisungen oder Beipackzettel.

### Der erste Code

Der erste Code ist eine digitale Kodierung, die auf der äußeren Verpackung abgedruckt wurde und die pZ kennzeichnet.

### Der zweite Code

Der zweite Code ist eine digitale Kodierung, die auf dem Behältnis abgedruckt wurde.

### Autorisierte Personen (aP)

Autorisierte Personen (aP) werden erfindungsgemäß Personen genannt, die berechtigt sind, die pZ an den Patienten herauszugeben, an den Patienten zu verschicken, oder die Behandlungsziele und -erfolge zu prüfen. AP können auch Personen sein, die die Einnahme der pZ in der Videoaufnahme validieren. Die aP sind z.B. Ärzte, Arztpraxispersonal, Apotheker, Krankenhauspersonal, Bewährungshelfer, künstliche Intelligenzen (KI) usw.

### Anwendungssoftware

Eine Anwendungssoftware ist ein Computerprogramm, das sich auf einem elektronischen Gerät befindet und das genutzt wird, um die gewünschte Funktionalität zu bearbeiten und zu unterstützen. Dieses Computerprogramm kann auch in Form einer Anwendungssoftware für mobile Geräte ("App" oder "mobile App") angeboten werden. Erfindungsgemäß unterstützt die Anwendungssoftware die Videoaufnahmen des Patienten und die Weiterleitung dieser Videoaufnahmen über eine Blockchain an die vorgegebene digitale Adresse. Darüber hinaus kann die Anwendungssoftware auch weitere Merkmale aufweisen, wie z.B. zeitliche Begrenzung der Videoaufnahmen, Gesichtserkennung, Erkennung der digitalen Codes usw.

### Adhärenz

Gemäß Wikipedia (https://de.wikipedia.org/wiki/Adhärenz) bezeichnet der Begriff Adhärenz das Ausmaß, in dem das Verhalten einer Person, wie die Medikamenteneinnahme, ein Diätregime oder eine Lebensstiländerung, mit den mit dem Therapeuten vereinbarten Empfehlungen übereinstimmt. Grundlage einer erfolgreichen Therapie ist - dieser Auffassung entsprechend - die Berücksichtigung der individuellen Bedürfnisse des Patienten sowie die Berücksichtigung von Faktoren, die es dem Patienten erschweren, das Therapieziel zu erreichen. Gute Adhärenz entspricht konsequentem Befolgen des mit dem Therapeuten vereinbarten Behandlungsplanes. Gemäß der Weltgesundheitsorganisation (WHO) erreichen im Durchschnitt nur 50 % der Patienten eine gute Adhärenz. Besonders wichtig ist die Adhärenz bei chronisch Kranken in Bezug auf die Einnahme von Medikamenten, oder etwa das Befolgen einer Diät oder die Veränderung des Lebensstils.

### Blockchain

Die Blockchain verknüpft die beteiligten Datensätze ("Blöcke"), sodass eine zusammenhängende Informationskette entsteht. Innerhalb der Blockchain wird jeder Block mit dem Block vor und nach ihm verbunden, sodass eine irreversible, nicht veränderbare Kette entsteht.

Durch die Verkettung der Blöcke wird verhindert, dass ein Block geändert, oder zwischen zwei vorhandenen Blöcken ein weiterer Block eingefügt wird.

Jeder Block enthält dabei typischerweise einen kryptographisch sicheren Hash (Streuwert) des vorhergehenden Blocks, einen Zeitstempel und Transaktionsdaten (s. https://de.wikipedia.org/wiki/Blockchain)

Die Blockchain kann in jedweder Anwendung eingesetzt werden. Für die Blockchain ist entscheidend, dass spätere Transaktionen und die Blöcke, die darauf basieren, auf früheren Transaktionen aufbauen und diese als richtig bestätigen, indem sie die Kenntnis der früheren Transaktionen beweisen. Damit wird es unmöglich gemacht, Existenz oder Inhalt der früheren Transaktionen zu manipulieren oder zu tilgen, ohne gleichzeitig alle späteren Transaktionen ebenfalls zu zerstören. Andere Teilnehmer der dezentralen Buchführung, die noch Kenntnis der späteren Transaktionen haben, würden eine manipulierte Kopie der Blockchain daran erkennen, dass sie Inkonsistenzen in den Berechnungen aufweist.

Erfindungsgemäß wird die Blockchain-Technologie benutzt, um den Weg einer pZ von der Herstellung, über die Abgabestelle zum Patienten und die nachweisliche Einnahme der pZ durch den Patienten manipulationssicher abzuspeichern und zu verifizieren.

### Elektronisches Gerät

Als elektronisches Gerät wird ein Gerät bezeichnet, welches funktional für die Nutzung der erfindungsgemäßen Anwendungssoftware geeignet ist und für die Sprach- und Datenkommunikation eingesetzt wird.

### Digitaler Zwilling

Ein digitaler Zwilling ist gemäß dem Wirtschaftslexikon Gabler ein virtuelles Modell z.B. eines Prozesses, eines Produkts oder einer Dienstleistung, welches die reale und virtuelle Welt verbindet.

Diese Kopplung der virtuellen und realen Welten ermöglicht die Analyse von Daten und die Überwachung von Systemen, sodass der gesamte Lebenszyklus eines Produkts erstellt werden kann.

Ein digitaler Zwilling kann erfindungsgemäß eingesetzt werden, um die pZ nachzubilden.

### GEBIET DER ERFINDUNG

Durch die Anwendung des erfindungsgemäßen Verfahrens ist eine lückenlose Protokollierung möglich, wann und was der Patient eingenommen hat, nämlich welche pZ und zum welchem Zeitpunkt diese pZ eingenommen wurde. Damit haben die aP, die die pZ verordnet hat, aber auch die Gesundheitssysteme die Möglichkeit, die verordnungsgemäße Einnahme der pZ ggf. auch auf Ebene der Einzeldosis zu verifizieren.

Die vorliegende Erfindung wird für die Verwendung im Rahmen der endogenen Markierung von Urinproben eingesetzt. Diese findet insbesondere zur Detektion von Substanzmissbrauch statt. Mögliche Anwendungsbereiche sind z. B. die Substitutionsbehandlung Opiatabhängiger, Drogentherapie, Dopingkontrolle und Abstinenzkontrolle. Beispielsweise in der Substitutionsbehandlung sind zwei Aspekte von besonderer Bedeutung:
- Einerseits muss sichergestellt werden, dass der Patient das Substitutionsmittel tatsächlich einnimmt und die Einnahme nicht vortäuscht, um das Substitutionsmittel ggf. zu verkaufen, und
- andererseits muss gefährdender Beikonsum ausgeschlossen werden, was meist durch Urinanalyse erfolgt.

### KURZE BESCHREIBUNG DER FIGUREN

Die vorliegende Erfindung ermöglicht eine lückenlose Protokollierung von der Herstellung bis zur Einnahme einer pZ. Diese Protokollierung erfolgt in einer Blockchain, in der alle relevanten Handlungen der am Prozess Beteiligten bezüglich dieser pZ registriert werden.

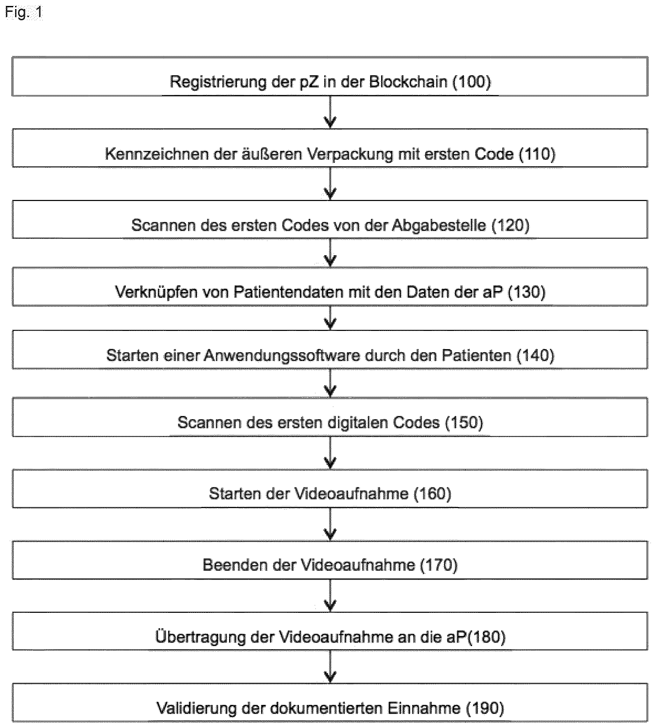

Eine schematische Darstellung der Erfindung ist in Fig. 1 wiedergegeben.

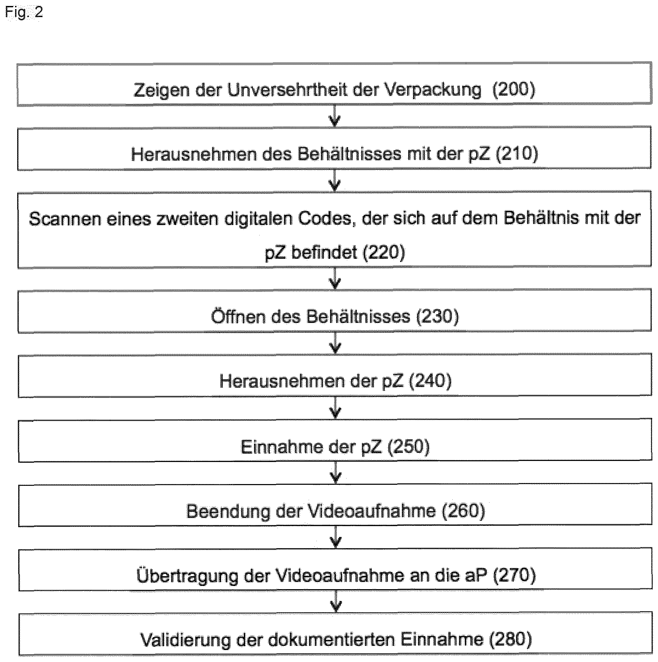

Eine Darstellung der Erfindung in der endogenen Markierung von Urinproben ist in Fig. 2 wiedergegeben.

### AUSFÜHRLICHE BESCHREIBUNG DER ERFINDUNG

Um den Fälschungsschutz zu gewährleisten, wird z.B. in Deutschland das an den EU-Hub angeschlossene securPharm-System verwendet. Dieses System basiert auf dem Ende-zu-Ende-Prinzip, bei dem die beiden Enden der Logistikkette zur Absicherung dienen (s. https://www.securpharm.de/sicherheitsmerkmale/). Das eine Ende ist der pharmazeutische Unternehmer (pU), der eine pZ in Verkehr bringt. Das andere Ende ist die Abgabestelle, etwa eine öffentliche Apotheke.

Die Verordnung (EU) 2016/161 (Fälschungsschutzrichtlinie) fordert zwei Sicherheitsmerkmale auf der Packung von verschreibungspflichtigen Human-Arzneimitteln:
- Ein Erstöffnungsschutz (Anti-tampering Device) über den erkennbar ist, ob die äußere Verpackung eines Arzneimittels unversehrt ist. Dieses Sicherheitsmerkmal muss von jedem pharmazeutischen Unternehmer selbst umgesetzt werden. Die DIN EN 16679 gibt dafür einen einheitlichen europäischen Standard vor.
- Ein individuelles Erkennungsmerkmal (Unique Identifier), das jede Packung zum Unikat und über den enthaltenen digitalen Produktcode eindeutig identifizierbar macht. Dieser Unique Identifier ist die Grundlage für die Echtheitsprüfung durch das securPharm-System.

Während des Produktionsprozesses versieht der pharmazeutische Unternehmer jede Packung mit den Sicherheitsmerkmalen. Die Daten des individuellen Erkennungsmerkmals (Seriennummer, Produktcode, Chargennummer, Verfalldatum) werden in Klarschrift und im Data Matrix Code auf die Packung aufgebracht und in die zentrale Datenbank der pharmazeutischen Industrie hochgeladen. Zur Echtheitsprüfung wird der Data Matrix Code vor der Abgabe an den Patienten gescannt. Dadurch werden die Daten der Packung mit den Daten im System abgeglichen. Der Status der Packung wird an die Apotheke zurückgemeldet, d.h. ob das individuelle Erkennungsmerkmal aktiviert oder bereits deaktiviert wurde. Ist letzteres der Fall, darf die Packung nicht an den Patienten abgegeben werden.

Es ist die Aufgabe der Erfindung, nicht nur den Hersteller und die Abgabestelle zu verifizieren, sondern die ganze Kette von der Herstellung der pZ bis zur Patienteneinnahme nachvollziehen zu können.

Die Erfindung wird anhand der Figuren näher erläutert.

Diese zeigen:
Fig. 1 die Schritte, die von der Herstellung bis zur Validierung der Einnahme durchgeführt werden;
Fig. 2 die Schritte, die für die Anwendung des erfindungsgemäßen Verfahrens bei der endogenen Markierung von Urinproben notwendig sind.

Die Fig. 1 zeigt die Schritte:
- Registrierung der pZ in der Blockchain (100)
- Kennzeichnen der äußeren Verpackung mit einem ersten Code (110);
- Scannen des ersten Codes von der Abgabestelle (120);
- Verknüpfen von Patientendaten mit den Daten der aP (130);
- Starten einer Anwendungssoftware durch den Patienten (140);
- Scannen des ersten digitalen Codes (140);
- Starten der Videoaufnahme (150);
- Beenden der Videoaufnahme (160);
- Übertragung der Videoaufnahme an die aP (180).
- Validierung der dokumentierten Einnahme (190)

In Schritt 100 der Fig. 1 wird als Teil des Herstellungsprozesses die pZ in der Blockchain registriert. Dies kann z. B. unter Verwendung von digitalen Zwillingen erfolgen.

Im Schritt 110 der Fig. 1 wird die äußere Verpackung der pZ mit einem ersten digitalen Code durch den Hersteller gekennzeichnet. Entsprechend dem securPharm-System werden die Daten des ersten Codes (Seriennummer, Produktcode, Chargennummer, Verfallsdatum) in Klarschrift und im digitalen Code auf die äußere Verpackung aufgebracht.

Im Schritt 120 wird zur Echtheitsprüfung der Code vor der Abgabe an den Patienten gescannt. Die Abgabestelle kann die Apotheke oder die aP sein.

Durch das Scannen des ersten Codes können die Patientendaten mit den Daten der aP im Schritt 130 verknüpft werden. Diese Verknüpfung kann durch das Scannen des ersten Codes automatisch erfolgen, oder durch die aP manuell erfolgen. Durch die Verknüpfung der Daten können die Videoaufnahmen des Patienten, die im Schritt 160 gemacht werden, an die richtige digitale Adresse der aP im Schritt 130 versendet werden.

Der Zeitpunkt beziehungsweise der Zeitrahmen, wann die pZ vom Patienten eingenommen werden soll, kann von der aP festgelegt werden.

Erfindungsgemäß ist auch vorgesehen, dass der Patient vor dem festgelegten Zeitpunkt eine elektronische Nachricht erhält, die ihn entweder anweist, wann die Einnahme erfolgen soll, oder ihn an die Einnahme erinnert. Die elektronische Nachricht kann z.B. eine E-Mail, eine SMS oder eine Push-Nachricht sein.

Vor der Einnahme der pZ startet der Patient eine Anwendungssoftware (Schritt 140), die auf einem elektronischen Gerät vorhanden ist. Das elektronische Gerät kann stationär (Computer oder Computerterminal) oder mobil (z.B. Mobiltelefon, Tablet oder Laptop) sein.

Nach dem Scannen des auf der äußeren Verpackung befindlichen ersten digitalen Codes durch den Patienten (150) kann der Patient die Videoaufnahme in der Anwendungssoftware starten (160), um die Einnahme der pZ nachzuweisen.

Durch das Starten der Anwendungssoftware kann automatisch eine verschlüsselte oder eine unverschlüsselte Datenübertragung aktiviert werden.

Für den Nachweis der Einnahme der pZ durch den Patienten soll die Videoaufnahme eine oder mehrere der folgenden Handlungen beinhalten, wie z.B. Zeigen des Gesichtes, Zeigen des Öffnens der äußeren Verpackung durch den Patienten, Zeigen der Herausnahme des Behältnisses aus der äußeren Verpackung, Zeigen der Herausnahme der pZ aus dem Behältnis, oder Zeigen der Einnahme der pZ durch den Patienten. Nach dem Beenden der Videoaufnahme (170) wird diese Videoaufnahme dann im Schritt 180 zur Validierung (190) an die aP verschickt. Nach erfolgter Validierung können bestimmte Informationen den Gesundheitssystemen zu Abrechnungszwecken zur Verfügung gestellt werden.

Die Videoaufnahmen werden durch eine aP validiert.

Eine weitere Aufgabe der Erfindung ist sicherzustellen, dass jede einzelne pZ als Einzeldosis von der Herstellung bis zur Verabreichung bzw. Einnahme nachverfolgt werden kann. Damit soll einerseits sichergestellt werden, dass die Authentizität der pZ sichergestellt wird, und andererseits, dass der Patient, dem diese pZ verschrieben wurde, diese auch tatsächlich eingenommen hat. Dafür wird auf dem Behältnis, in dem sich die einzelne pZ befindet, ein zweiter Code angebracht. Das bedeutet, dass jeder einzelnen pZ ein Code zugeordnet wird. Die pZ muss daher einzeln umhüllt werden. Die Umhüllung der pZ ist mit einem Code versehen. Es ist aber nicht ausgeschlossen, dass mehrere pZ zusammen z.B. in einer Blisterpackung vorliegen. Allerdings muss dann sichergestellt werden, dass jeder pZ ein eindeutiger Code zugeordnet wird.

Diese Ausführungsform ist besonders dann von Vorteil, wenn die aP befürchten muss, dass der Patient nicht beabsichtigt, die pZ einzunehmen. Dieses Verhalten wird bei Substanzmissbrauch und der endogenen Markierung von Urinproben oft beobachtet.

In der Substanzmissbrauchskontrolle ist sehr wichtig, jegliche Manipulationsmöglichkeit einer Urinprobe zu vermeiden. Zu diesem Zweck werden Urin-Marker eingesetzt. Die äußere Verpackung der Urin-Marker für die gesicherte Urinkontrolle ist so gestaltet, dass mehrere Manipulationsschutzmerkmale während des Öffnungsprozesses greifen. Die Anwendungssoftware fordert den Nachweis dieser Manipulationsschutzmerkmale zu definierten Prozessschritten während der Einnahme des Urin-Markers zur Sicherstellung einer eindeutigen Zuordnung des Urins zum Patienten. Erfolgt dieser Nachweis nicht, wird die aktuelle Sitzung automatisch beendet und das Sitzungsfragment an die aP zur Validierung weitergeleitet.

Diese Herangehensweise kann sowohl synchron, also bei zeitgleicher Kommunikation zwischen Patient und aP im Rahmen einer virtuellen Sprechstunde, als auch asynchron erfolgen, d. h. mit zeitlichem Versatz, sodass die Aufnahme zu einem anderen Zeitpunkt erfolgt als die Validierung der Videoaufnahme durch die aP. Damit ist es möglich, eine zeit- und ortsunabhängige Einnahme, bei der eine Manipulation der pZ ausgeschlossen ist, zu gewährleisten.

Beispielsweise bei der Substitutionstherapie Drogenabhängiger wird der Erfolg der Therapie durch die Urinkontrolle bestimmt. Das Bestreben, einen eventuellen Rückfall in den Substanzmissbrauch aus Furcht vor Sanktionen zu verschleiern, ist daher zu erwarten.

Im Rahmen einer solchen Substitutionstherapie enthält die pZ für die Urinauthentifizierung durch eine chromatographische Analyse, die im erfindungsgemäßen Verfahren eingesetzt wird, keinen Wirkstoff, sondern eine Kombination der in der Regel nicht metabolisierbaren Markersubstanzen, die die Verfälschung der Urinproben ausschließen soll. Wird die Markersubstanz nicht zuverlässig eingenommen, kann diese zu einem späteren Zeitpunkt einer anderen, drogenfreien Person ausgehändigt werden, um dann deren Urin zur Untersuchung einzusenden. Das Verfahren zur Verwendung von Markern und insbesondere Polyethylen-Glycolen mit verschiedenen Molekulargewichten, um die Manipulation von Urinproben zu verhindern, ist in EP 1 410 014 beschrieben. Die Verwendung von Kapseln, die die Markersubstanzen enthalten, ist in EP 2 957 909 beschrieben.

Die Aufgabe der Erfindung wird dadurch gelöst, dass jede Einzeldosis einer pZ bei deren Herstellung und anschließender Verpackung in einer Blockchain registriert wird.

Die pZ werden in einer Ausführung bei der endogenen Markierung der Urinproben an Labore verschickt, welche ihrerseits Einrichtungen von aP beliefern können. In einer anderen Ausführung werden die aP unmittelbar beliefert. Die Patienten erhalten die pZ direkt von den aP. Im Normalfall kennt die aP die Zusammensetzung der Markersubstanzen nicht. Die Blockchain verknüpft die beteiligten Einheiten, sodass eine zusammenhängende Informationskette entsteht. Damit kann das Labor die Identität der pZ verifizieren.

Bei der Anwendung des erfindungsgemäßen Verfahrens bei der endogenen Markierung der Urinprobe z.B. in der Substitutionstherapie, muss die Einnahme der pZ vom Patienten durch genau festgelegte Schritte erfolgen, die sicherstellen, dass die Markerkombination vom Patienten, der die Urinprobe abgibt, tatsächlich eingenommen wurde. Dafür sind weitere Schritte nach dem Starten der Videoaufnahme vor der Einnahme der pZ vorgesehen, wie in Fig. 2 beschrieben:
- Zeigen der Unversehrtheit der Verpackung (200)
- Herausnehmen des Behältnisses mit der pZ (210)
- Scannen eines zweiten digitalen Codes, der sich auf dem Behältnis mit der pZ befindet (220);
- Öffnen des Behältnisses (230);
- Herausnehmen der pZ (240);
- Einnahme der pZ (250);
- Beendung der Videoaufnahme; (260)
- Übertragung der Videoaufnahme an die aP (270)
- Validierung der dokumentierten Einnahme (280)

Durch das Scannen des zweiten digitalen Codes, der sich auf dem Behältnis mit der pZ befindet, (220) wird ein Zeitfenster gestartet. Das bedeutet, dass das Scannen des zweiten Codes der Startpunkt dieser bestimmten Zeitspanne ist, innerhalb welcher die Schritte des erfindungsgemäßen Verfahrens durchgeführt werden müssen. Diese Maßnahme dient dazu, eventuelle Manipulationen zu verhindern. Falls in diesem Zeitfenster die Einnahme nicht erfolgt, wird die aP informiert. Es kann vorgesehen werden, dass eine Wiederholung der Videoaufnahme mit derselben pZ nicht möglich ist und der Patient in so einem Fall einen neunen Termin mit der aP vereinbaren muss.

In einer Ausführungsform beträgt das Zeitfenster bis zu 120 Sek. In einer andere Ausführungsform beträgt das Zeitfenster 30, 45, 60, 75, 90 oder 120 Sek.

Auf dem Behältnis kann sich ein weiterer abnehmbarer Code befindet, der zur Kennzeichnung der Urinprobe vor dem Versand an das Labor dient.

Insbesondere bei der endogenen Markierung der Urinprobe kann die Einnahme von Produkten ggf. nur vorgetäuscht werden, um diese zu einem späteren Zeitpunkt weiterzugeben. Für diesen Fall kann der pZ ein Farbstoff zugesetzt werden, der die Mundschleimhaut färbt, sofern die pZ nicht geschluckt wird, sondern eine gewisse Zeit im Mund verbleibt.

Falls gewünscht kann im erfindungsgemäßen Verfahren daher noch einen Schritt vorgesehen werden, nämlich die Überprüfung des Mundraums nach der erfolgten Einnahme.

In einer Ausführungsform ist der erste Code ein eindimensionaler oder mehrdimensionaler Code.

In einer weiteren Ausführungsform ist der zweite Code ein zweidimensionaler Code.

In einer weiteren Ausführungsform sind der erste Code und der zweite Code zweidimensionale Codes.

In einer weiteren Ausführungsform sind die zweidimensionale Codes QR-Codes.

Das erfindungsgemäße Verfahren kann für verschieden Zwecke einsetzt werden.

Die Anwendungssoftware kann aber auch vom Gesundheitssystem an die aP zu Abrechnungszwecken zur Verfügung gestellt werden. Damit kann ermöglicht werden, dass die Patienten, die eine gute Adhärenz aufweisen, z. B. einen Rabatt bei der Abrechnung erhalten.

Darüber hinaus kann das erfindungsgemäße Verfahren bei der endogenen Markierung der Urinprobe eingesetzt werden, um die ortsunabhängige Einnahme der Markersubstanzen zu verifizieren. Damit kann sichergestellt werden, dass die Urinprobe vom bestimmten Patienten stammt, ohne die Notwendigkeit den Patienten in die Praxis bestellen zu müssen.

Die Erfindung umfasst außerdem eine Anwendungssoftware, die auf einem durch einen Computer lesbaren Medium gespeichert ist und die einen Prozessor zum Scannen eines ersten Codes auf der äußeren Verpackung einer pZ, zum Empfangen von Daten aus einer Blockchain, zum Durchführen einer Videoaufnahme und zur Übertragung der Videoaufnahme an die Blockchain befähigt. Die Anwendungssoftware ist so konfiguriert, dass sie eine Schnittstelle mit der Blockchain aufweist.

Die Anwendungssoftware ist vorzugsweise auf einem mobilen Endgerät gespeichert. Das mobile Endgerät ist vorzugsweise ein Telefon, ein Tablet oder ein Laptop.

## Patentansprüche

1. Verfahren zur Verwendung einer pharmazeutischen Zusammensetzung bei der endogenen Markierung einer Urinprobe, wobei die pharmazeutische Zusammensetzung keinen Wirkstoff, sondern nur eine Kombination aus mindestens zwei Markersubstanzen enthält und wobei die Markersubstanzen Polyethylenglycole mit verschiedenen Molekulargewichten sind, **gekennzeichnet durch** folgende Schritte:
- Registrierung jeder Einzeldosis der pharmazeutischen Zusammensetzung bei deren Herstellung und anschließender Verpackung in einer Blockchain (100), wobei in der Blockchain alle relevanten Handlungen der an dem Prozess Beteiligten bezüglich dieser pharmazeutischen Zusammensetzung registriert werden zur lückenlosen Protokollierung von der Herstellung bis zur Einnahme der pharmazeutischen Zusammensetzung;
- Kennzeichnen einer äußeren Verpackung mit einem ersten digitalen Code (110);
- Scannen des ersten digitalen Codes von einer Abgabestelle (120);
- Verknüpfen von Patientendaten eines Patienten mit Daten einer autorisierten Person (130), wobei die Blockchain die beteiligten Einheiten verknüpft, sodass eine zusammenhängende Informationskette entsteht;
- Starten einer Anwendungssoftware durch den Patienten (140);
- Scannen des ersten digitalen Codes durch die Anwendungssoftware (150);
- Starten einer Videoaufnahme (170);
- Zeigen einer Unversehrtheit der äußeren Verpackung (200);
- Herausnehmen eines Behältnisses mit der pharmazeutischen Zusammensetzung aus der äußeren Verpackung (210);
- Scannen eines zweiten digitalen Codes, der sich auf dem Behältnis mit der pharmazeutischen Zusammensetzung befindet (220);
- Öffnen des Behältnisses (230);
- Herausnehmen der pharmazeutischen Zusammensetzung aus dem Behältnis (240);
- Einnahme der pharmazeutischen Zusammensetzung (250);
- Beendung der Videoaufnahme (260);
- Übertragung der Videoaufnahme an die autorisierte Person und durch die Anwendungssoftware an die Blockchain (270);
- Validierung der dokumentierten Einnahme durch die autorisierte Person (280).

2. Verfahren nach Anspruch 1, **gekennzeichnet durch** eine Mundraumkontrolle, die nach der Einnahme der pharmazeutischen Zusammensetzung erfolgt.

3. Verfahren nach einem der Ansprüche 1 oder 2, **gekennzeichnet dadurch, dass** der Zeitpunkt der Durchführung des Verfahrens von der autorisierten Person festgelegt wird.

4. Verfahren nach Anspruch 3, **gekennzeichnet dadurch, dass** der Patient einen Tag vor dem festgelegten Datum für die Durchführung des Verfahrens eine elektronische Nachricht erhält.

5. Verfahren nach Anspruch 1, **gekennzeichnet dadurch, dass** durch das Scannen des zweiten Codes ein Zeitfenster gestartet wird.

6. Verfahren nach Anspruch 5, **gekennzeichnet dadurch, dass** das Zeitfenster bis zu 120 Sekunden beträgt.

7. Verfahren nach einem der Ansprüche 5 oder 6, **gekennzeichnet dadurch, dass** der Patient bei Überschreitung des Zeitfensters einen neuen Termin mit der autorisierten Person vereinbaren muss.

8. Verfahren nach einem der Ansprüche 1 bis 4, **gekennzeichnet dadurch, dass** durch das Scannen des ersten Codes eine verschlüsselte Datenübertragung aktiviert wird.

9. Verfahren nach einem der Ansprüche 1 bis 5, **gekennzeichnet dadurch, dass** das elektronische Gerät ein mobiles elektronisches Gerät ist.

10. Verfahren nach einem der Ansprüche 1 bis 6, **gekennzeichnet dadurch, dass** die pharmazeutische Zusammensetzung in Form einer Kapsel ist.

11. Verfahren nach einem der Ansprüche 1 bis 6, **gekennzeichnet dadurch, dass** die pharmazeutische Zusammensetzung in Form einer Tablette ist.

12. Verfahren nach einem der Ansprüche 1 bis 6, **gekennzeichnet dadurch, dass** die pharmazeutische Zusammensetzung parenteral verabreicht wird.

13. Äußere Verpackung mit einem darin angeordneten Behältnis mit einer pharmazeutischen Zusammensetzung zur Verwendung in dem Verfahren nach einem der Ansprüche 1 bis 12, wobei die äußere Verpackung mit einem ersten digitalen Code gekennzeichnet ist, wobei die äußere Verpackung so gestaltet ist, dass mehrere Manipulationsschutzmerkmale während eines Öffnungsprozesses greifen, wobei das Behältnis mit aufweisend einem zweiten digitalen Code gekennzeichnet ist, wobei die pharmazeutische Zusammensetzung keinen Wirkstoff, sondern nur eine Kombination aus mindestens zwei Markersubstanzen enthält und wobei die Markersubstanzen Polyethylenglycole mit verschiedenen Molekulargewichten sind.

14. Anwendungssoftware, die auf einem durch einen Computer lesbaren Medium gespeichert ist und, die einen Prozessor zur Durchführung der folgenden Schritte des Verfahrens nach einem der Ansprüche 1 bis 12 befähigt:
- Scannen eines ersten digitalen Codes auf einer äußeren Verpackung mit einem darin angeordneten Behältnis mit einer pharmazeutischen Zusammensetzung, wobei die pharmazeutische Zusammensetzung keinen Wirkstoff, sondern nur eine Kombination aus mindestens zwei Markersubstanzen enthält und wobei die Markersubstanzen Polyethylenglycole mit verschiedenen Molekulargewichten sind;
- Empfangen von Daten aus einer Blockchain, wobei in der Blockchain alle relevanten Handlungen der an dem Prozess Beteiligten bezüglich dieser pharmazeutischen Zusammensetzung registriert werden zur lückenlosen Protokollierung von der Herstellung bis zur Einnahme der pharmazeutischen Zusammensetzung, wobei die Blockchain die beteiligten Einheiten verknüpft, sodass eine zusammenhängende Informationskette entsteht;
- Ermöglichen einer Videoaufnahme, von:
∘ Zeigen einer Unversehrtheit der Verpackung (200);
∘ Herausnehmen des Behältnisses mit der pharmazeutischen Zusammensetzung aus der äußeren Verpackung (210);
∘ Scannen eines zweiten digitalen Codes, der sich auf dem Behältnis mit der pharmazeutischen Zusammensetzung befindet (220),
∘ Öffnen des Behältnisses (230);
∘ Herausnehmen der pharmazeutischen Zusammensetzung aus dem Behältnis (240);
∘ Einnahme der pharmazeutischen Zusammensetzung (250);
- Übertragung der Videoaufnahme an die Blockchain (270).

15. Ein mobiles Gerät enthaltend eine Anwendungssoftware nach Anspruch 14.

## Claims

1. A method for using a pharmaceutical composition for endogenous urine sample marking, wherein the pharmaceutical composition contains no active ingredient, but merely a combination of at least two marker substances, and wherein the marker substances are polyethylene glycols with different molecular weights, **characterised by** the following steps:
- registering, in a blockchain (100), each individual dose of the pharmaceutical composition during its preparation and subsequent packaging, wherein all relevant actions of the parties involved in the process regarding this pharmaceutical composition are registered in the blockchain for complete logging from the time of preparation to the time of ingestion of the pharmaceutical composition;
- marking an outer packaging with a first digital code (110);
- scanning the first digital code from a dispensary (120);
- linking patient data of a patient with data of an authorised person (130), wherein the blockchain links the units involved so that a coherent information chain is created;
- starting an application software by the patient (140);
- scanning the first digital code by the application software (150);
- starting a video capture (170);
- demonstrating that the outer packaging (200) is intact;
- removing a container containing the pharmaceutical composition from the outer packaging (210);
- scanning a second digital code, which is located on the container containing the pharmaceutical composition (220);
- opening the container (230);
- removing the pharmaceutical composition from the container (240);
- ingesting the pharmaceutical composition (250);
- ending the video capture (260);
- transmitting the video capture to the authorised person and by the application software to the blockchain (270);
- validating the documented ingestion by the authorised person (280).

2. The method according to claim 1, **characterised by** a check of the oral cavity, which is performed following ingestion of the pharmaceutical composition.

3. The method according to any one of claims 1 or 2, **characterised in that** the time at which the method is performed by the authorised person is specified.

4. The method according to claim 3, **characterised in that** the patient receives an electronic message a day before the date specified for performing the method.

5. The method according to claim 1, **characterised in that** a time window is started by the scanning of the second code.

6. The method according to claim 5, **characterised in that** the time window is up to 120 seconds.

7. The method according to any one of claims 5 or 6, **characterised in that** the patient must make a new appointment with the authorised person if the time window is exceeded.

8. The method according to any one of claims 1 to 4, **characterised in that** an encrypted data transfer is activated by the scanning of the first code.

9. The method according to any one of claims 1 to 5, **characterised in that** the electronic device is a mobile electronic device.

10. The method according to any one of claims 1 to 6, **characterised in that** the pharmaceutical composition is in the form of a capsule.

11. The method according to any one of claims 1 to 6, **characterised in that** the pharmaceutical composition is in the form of a tablet.

12. The method according to any one of claims 1 to 6, **characterised in that** the pharmaceutical composition is administered parenterally.

13. An outer packaging with a container arranged therein containing a pharmaceutical composition for use in the method according to any one of claims 1 to 12, wherein the outer packaging is marked with a first digital code, wherein the outer packaging is formed such that a plurality of anti-tampering features are effective during an opening process, wherein the container is marked by a second digital code, wherein the pharmaceutical composition does not contain an active ingredient, but merely a combination of at least two marker substances, and wherein the marker substances are polyethylene glycols having different molecular weights.

14. An application software which is stored on a medium readable by a computer and allows a processor to perform the following steps of the method according to one of claims 1 to 12:
- scanning a first digital code on an outer packaging with a container arranged therein containing a pharmaceutical composition, wherein the pharmaceutical composition does not contain an active ingredient, but merely a combination of at least two marker substances, and wherein the marker substances are polyethylene glycols having different molecular weights;
- receiving data from a blockchain, wherein all relevant actions of the parties involved in the process regarding this pharmaceutical composition are registered in the blockchain for complete logging from the time of preparation to the time of ingestion of the pharmaceutical composition; wherein the blockchain links the units involved so that a coherent information chain is created;
- allowing a video capture of
∘ demonstration that the outer packaging (200) is intact;
∘ removal of the container containing the pharmaceutical composition from the outer packaging (210);
∘ scanning of a second digital code, which is located on the container containing the pharmaceutical composition (220);
∘ opening of the container (230);
∘ removal of the pharmaceutical composition from the container (240);
∘ ingestion of the pharmaceutical composition (250);
- transmitting the video capture to the blockchain (270).

15. A mobile device containing an application software according to claim 14.

## Revendications

1. Procédé d'utilisation d'une composition pharmaceutique lors d'un marquage endogène d'un échantillon d'urine, dans lequel la composition pharmaceutique ne contient aucune substance active mais uniquement une combinaison à base d'au moins deux substances de marquage et dans lequel les substances de marquage sont des poly éthylène glycols avec des poids moléculaires différents, **caractérisé par** les étapes suivantes :
- l'enregistrement de chaque dose individuelle de la composition pharmaceutique lors de sa fabrication et de son emballage ultérieur dans une chaine de blocs (100), où, tous les actes pertinents des participants au processus concernant cette composition pharmaceutique sont enregistrés dans la chaine de blocs pour une consignation sans faille allant de la fabrication à l'administration de la composition pharmaceutique ;
- le marquage de l'emballage extérieur avec un premier code numérique (110) ;
- le balayage du premier code numérique par un point de distribution (120) ;
- la combinaison de données de patient d'un patient avec des données d'une personne autorisée (130), où la chaine de blocs combine les unités impliquées de sorte qu'une chaine d'information correspondante se trouve créée ;
- le démarrage d'un logiciel d'application par le patient (140) ;
- le balayage du premier code numérique par le logiciel d'application (150) ;
- le démarrage d'un enregistrement vidéo (170) ;
- l'indication d'une inviolabilité de l'emballage extérieur (200) ;
- la sortie d'un récipient avec la composition pharmaceutique hors de l'emballage extérieur (210) ;
- le balayage d'un deuxième code numérique qui se trouve sur le récipient avec la composition pharmaceutique (220) ;
- l'ouverture du récipient (230) ;
- la sortie de la composition pharmaceutique hors du récipient (240) ;
- l'administration de la composition pharmaceutique (250) ;
- la fin de l'enregistrement vidéo (260) ;
- la transmission de l'enregistrement vidéo à la personne autorisée et par le logiciel d'application à la chaîne de blocs (270) ;
- la validation de l'administration documentée par la personne autorisée (280).

2. Procédé selon la revendication 1, **caractérisé par** un contrôle de la cavité buccale qui a lieu après l'administration de la composition pharmaceutique.

3. Procédé selon l'une des revendications 1 ou 2, **caractérisé en ce que** le moment de l'exécution du procédé est fixé par la personne autorisée.

4. Procédé selon la revendication 3, **caractérisé en ce que** le patient reçoit un message électronique un jour avant la date fixée pour l'exécution du procédé.

5. Procédé selon la revendication 1, **caractérisé en ce qu'**un intervalle temporel est démarré par le balayage du deuxième code.

6. Procédé selon la revendication 5, **caractérisé en ce que** l'intervalle temporel dure jusqu'à 120 secondes.

7. Procédé selon l'une des revendications 5 ou 6, **caractérisé en ce que** le patient doit prendre un nouveau rendez-vous avec la personne autorisée en cas de dépassement de l'intervalle temporel.

8. Procédé selon l'une des revendications 1 à 4, **caractérisé en ce qu'**une transmission de données chiffrée est activée par le balayage du premier code.

9. Procédé selon l'une des revendications 1 à 5, **caractérisé en ce que** l'appareil électronique est un appareil électronique mobile.

10. Procédé selon l'une des revendications 1 à 6, **caractérisé en ce que** la composition pharmaceutique est sous la forme d'une gélule.

11. Procédé selon l'une des revendications 1 à 6, **caractérisé en ce que** la composition pharmaceutique est sous la forme d'un comprimé.

12. Procédé selon l'une des revendications 1 à 6, **caractérisé en ce que** la composition pharmaceutique est administrée par voie parentérale.

13. Emballage extérieur avec un récipient y étant disposé avec une composition pharmaceutique pour l'emploi dans le procédé selon l'une des revendications 1 à 12, où l'emballage extérieur est **caractérisé par** un premier code numérique, où l'emballage extérieur est conçu de telle manière que plusieurs caractéristiques de protection manipulatoires entrent en jeu pendant un processus d'ouverture, dans lequel le récipient est caractérisé en présentant un deuxième code numérique, dans lequel la composition pharmaceutique ne contient aucune substance active mais uniquement une combinaison à base d'au moins deux substances de marquage et dans lequel les substances de marquage sont des poly éthylène glycols avec des poids moléculaires différents.

14. Logiciel d'application, qui est enregistré sur un support lisible par un ordinateur, et qui active un processeur pour l'exécution des étapes suivantes du procédé selon l'une des revendications 1 à 12 :
- le balayage d'un premier code numérique sur un emballage extérieur avec un récipient y étant disposé avec une composition pharmaceutique, où la composition pharmaceutique ne contient aucune substance active mais uniquement une combinaison à base d'au moins deux substances de marquage et dans lequel les substances de marquage sont des poly éthylène glycols avec des poids moléculaires différents ;
- la réception de données à partir d'une chaine de blocs, où tous les actes pertinents des participants au processus concernant cette composition pharmaceutique sont enregistrés dans la chaine de blocs pour une consignation sans faille allant de la fabrication à l'administration de la composition pharmaceutique, où la chaine de blocs combine les entités impliquées de sorte qu'une chaine d'information correspondante est créée ;
- la possibilité d'un enregistrement vidéo de :
∘ l'indication d'une inviolabilité de l'emballage extérieur (200) ;
∘ la sortie d'un récipient avec la composition pharmaceutique hors de l'emballage extérieur (210) ;
o le balayage d'un deuxième code numérique qui se trouve sur le récipient avec la composition pharmaceutique (220) ;
∘ l'ouverture du récipient (230) ;
∘ la sortie de la composition pharmaceutique hors du récipient (240) ;
∘ l'administration de la composition pharmaceutique (250) ;
- la transmission de l'enregistrement vidéo à la chaîne de blocs (270).

15. Appareil mobile contenant un logiciel d'application selon la revendication 14.
